# EUROPEAN PATENT SPECIFICATION

(11) **EP 4 112 008 B1**
(45) Date of publication and mention of the grant of the patent: **22.04.2026**
(21) Application number: 21920711.5
(22) Date of filing: 18.11.2021
(51) Int. Cl.: A61F 2/24

(54) **INTERVENTIONAL ARTIFICIAL HEART VALVE AND MEDICAL APPARATUS**
INTERVENTIONELLE KÜNSTLICHE HERZKLAPPE UND MEDIZINISCHE VORRICHTUNG
PROTHÈSE VALVULAIRE CARDIAQUE INTERVENTIONNELLE ET APPAREIL MÉDICAL

(30) Priority: 20.01.2021 CN 202110076939; 20.01.2021 CN 202110075372
(43) Date of publication of application: 04.01.2023
(73) Proprietor: Shanghai Newmed Medical Co., Ltd., Shanghai 201321 (CN)
(72) Inventor: YU, Qifeng, Shanghai 201321 (CN); QIN, Tao, Shanghai 201321 (CN)
(74) Representative: Worthington, Richard Easton
(86) International application number: PCT/CN2021/131387
(87) International publication number: WO 2022/156335

(56) References cited:
- WO-A1-2017/151566
- WO-A1-2020/073056
- CN-A- 107 613 907
- CN-A- 107 690 323
- CN-A- 111 772 879
- CN-A- 112 089 506
- CN-A- 112 754 731
- CN-A- 112 754 732
- CN-U- 212 066 943
- US-A1- 2011 208 297
- US-A1- 2014 114 402
- US-A1- 2015 272 737
- US-A1- 2017 079 785

## Description

### Technical field

The present invention relates to the field of medical instruments, in particular to an interventional artificial heart valve and a medical apparatus for improving the function of a heart valve.

### Background Art

Heart valves are membrane structures that can be opened and closed in the organs of human or some animals. There are four valves in the heart of everybody, namely, an aortic valve connecting the left ventricle and the aorta, a pulmonary valve connecting the right ventricle and the pulmonary artery, a mitral valve connecting the left atrium and the left ventricle, and a tricuspid valve connecting the right atrium and the right ventricle. All of them act as one-way valves that enable the blood to flow from one direction to another direction but prevent the blood from flowing in the reversed direction. Mitral regurgitation results in myocardial remodeling and progressive ventricular enlargement, and eventually results in heart failure. In the prior art, the transcatheter mitral valve replacement (TMVR) surgery employs a catheter intervention approach, in which an artificial valve is pressed into a delivery system in vitro, delivered to the annulus of mitral valve in a human body, and released and fixed at the annulus of mitral valve to replace the native valve. Compared with surgical operations, TMVR does not require any auxiliary device for extracorporeal circulation and causes a smaller wound, the patient recovers more quickly after TMVR, and the hemodynamic indexes of the patient can be significantly improved after TMVR.

However, how to effectively fix the mitral valve or tricuspid valve in the heart and eliminate blood regurgitation is a technical problem to be solved urgently in the field of interventional artificial heart valves An interventional artificial heart valve in which a valve stent is handed to the valve leaflets by annular supporting elements has been reported. Such an interventional artificial heart valve can hold the valve leaflet tissue well and eliminate blood regurgitation after the implantation, but the use of barbs, for example, will destroy the normal physiological function of human valve leaflets after the implantation.

There are also anchoring structures without barbs in the art, which can reduce the damage to the valve leaflet tissue. However, it is very difficult to accurately deploy the closed ring elements of such an interventional artificial heart valve to the back of chordae tendineae and valve leaflets, and it is difficult to stably fix the interventional artificial heart valve after it is implanted, and there is a high probability of perivalvular leakage to different degrees. In addition, since the valve stent extends into the ventricular cavity, it may cause ventricular outflow tract obstruction to a certain degree, lead to complications such as arrhythmia and heart failure, and have severe adverse impacts on the rehabilitation process of the patient undergoing artificial valve replacement.

WO2020/073056 A1 describes a modular valve replacement system for treating valve-related cardiac disorders

### Summary of the Invention

The present invention discloses an interventional artificial heart valve according to claims 1 to 14 in order to solve the technical problems existing in the prior art.

In one aspect, the present invention provides an interventional artificial heart valve, which comprises a valve stent and an anchoring part, wherein the valve stent has a body section and a skirt section at an inflow end; a valve leaflet part is arranged in the body section; the valve stent can radially collapse and expand between a radially collapsed configuration and a radially expanded configuration; at least a part of the outer surface of the skirt section is covered by a flexible annular seal, the flexible annular seal comprises a material capable of absorbing blood; the material capable of absorbing blood expands after it absorbs blood; at least two connecting elements are arranged at a side of an outflow end of the body section; the anchoring part is arranged around the periphery of the valve stent and detachably connected to the connecting elements; and the anchoring part comprises at least two anchoring elements.

Preferably, the material capable of absorbing blood is one or more of PET fibers, polyester fibers and polyamide fibers.

Preferably, the flexible annular seal further covers a partial area of the body section at the side of the inflow end.

Preferably, the body section comprises a restraining part, the restraining part is arranged at the edge of the body section at the side of the inflow end to restrain the flexible annular seal from intruding into an internal space inside the valve stent.

Preferably, the anchoring element has an arc-shaped section, a free end of the arc-shaped section has a connecting part.

Preferably, both the arc-shaped section and the connecting part of the anchoring element are hollow tubular elements.

Preferably, the anchoring element has an arc-shaped section, an auxiliary section and a connecting part, wherein the connecting part is arranged at a free end of the arc-shaped section, and can be detachably connected to the connecting elements; and the auxiliary section is connected to the arc-shaped section to restrain the heart valve in the human body.

Preferably, the anchoring part can be connected with the connecting element, and is arranged around the periphery of the valve stent after it is connected with the connecting element, and the auxiliary section is arranged near the outflow end and near the side of the body section with respect to the arc-shaped section.

Preferably, the body section comprises a plurality of supporting parts arranged circumferentially; and the supporting parts extend radially outward and towards the inflow end.

Preferably, the supporting part comprises a first band of supporting part and a second band of supporting part that are arranged in the circumferential direction, wherein the first band of supporting part is closer to the side of the inflow end than the second band of supporting part.

Preferably, the arc-shaped section is located in an area that is generally enclosed by the first band of supporting part; and the auxiliary section is located in an area that is generally enclosed by the first band of supporting part or the second band of supporting part.

Preferably, the auxiliary section comprises an arc-shaped auxiliary section and connecting sections arranged at two ends of the arc-shaped auxiliary section, and the connecting sections are connected with the arc-shaped section; and the auxiliary section is located in an area that is generally enclosed by the first band of supporting part.

Preferably, the arc-shaped section comprises a plurality of separate sections, and the auxiliary section comprises a plurality of separate sections; the separate sections of the arc-shaped section and the separate sections of the auxiliary section are connected together by a plurality of connecting sections; the arc-shaped section, the auxiliary section, and the connecting sections are hollow tubular elements; and the auxiliary section is located in an area that is generally enclosed by the first band of supporting part.

Preferably, the auxiliary section comprises an arc-shaped auxiliary section and connecting sections arranged at two ends of the arc-shaped auxiliary section, and the connecting sections are connected with the arc-shaped section; and the auxiliary section is located in an area that is generally enclosed by the second band of supporting part.

Preferably, both the arc-shaped section and the connecting part of the anchoring element are hollow tubular elements.

Preferably, the first band of supporting part and/or the second band of supporting part have a convergent part near each of the two connecting elements, and a supporting part of the convergent part is more convergent radially than other supporting parts of the first band of supporting part and the second band of supporting part.

Preferably, a first connecting element and a second connecting element are provided at the edge of the body section at the side of the outflow end, the first connecting element is provided with a first connecting column and a second connecting column, and the second connecting element is provided with a third connecting column and a fourth connecting column; the anchoring part comprises a first anchoring element and a second anchoring element, a connecting part of the first anchoring element comprises a first socket and a second socket, and a connecting part of the second anchoring element comprises a third socket and a fourth socket; and the first socket, the second socket, the third socket and the fourth socket can receive the first connecting column, the third connecting column, the second connecting column and the fourth connecting column respectively.

Preferably, the first socket, the second socket, the third socket and the fourth socket and/or the first connecting column, the second connecting column, the third connecting column and the fourth connecting column have an anti-loose structure respectively.

Preferably, the anti-loose structure comprises one or more of pawl, ratchet, groove, flange and snap.

Preferably, all of the first connecting column, the second connecting column, the third connecting column and the fourth connecting column are hollow columns.

In another aspect, the present invention provides an interventional artificial heart valve, which comprises a valve stent and an anchoring part, wherein the valve stent has a body section and a skirt section at an inflow end; a valve leaflet part is arranged in the body section; the valve stent can radially collapse and expand between a radially collapsed configuration and a radially expanded configuration; at least two connecting elements are arranged at a side of an outflow end of the body section; the body section comprises a plurality of supporting parts arranged circumferentially; at least a part of the supporting parts extend radially outward and towards the inflow end; the anchoring part comprises at least two anchoring elements; the anchoring element has an arc-shaped section, an auxiliary section and a connecting part, wherein the connecting part is arranged at a free end of the arc-shaped section, and the connecting part can be detachably connected to the connecting elements; and the auxiliary section is connected to the arc-shaped section to restrain the heart valve in the human body; the anchoring part can be connected with the connecting element, and the anchoring part is arranged around the periphery of the valve stent after it is connected with the connecting element, and the auxiliary section is arranged near the outflow end and near the side of the body section with respect to the arc-shaped section.

In yet another aspect, the present invention provides a medical apparatus for improving the function of a heart valve, which comprises a valve stent and an anchoring part, wherein the valve stent has a body section and a skirt section at an inflow end; a valve leaflet part is arranged in the body section; the valve stent can radially collapse and expand between a radially collapsed configuration and a radially expanded configuration; at least a part of the outer surface of the skirt section is covered by a flexible annular seal, the flexible annular seal comprises a material capable of absorbing blood; the material capable of absorbing blood expands after it absorbs blood; at least two connecting elements are arranged at a side of an outflow end of the body section; the valve stent can be placed in a catheter in a radially collapsed state; the anchoring part comprises at least two anchoring elements, each of the two anchoring elements comprises an arc-shaped section and a connecting part arranged at a free end of the arc-shaped section; the connecting part can be detachably connected to the connecting elements; and the anchoring part has elasticity and can be placed in a catheter after elastic deformation.

Preferably, the anchoring element has an arc-shaped section, an auxiliary section and a connecting part, wherein the connecting part is arranged at a free end of the arc-shaped section, and can be detachably connected to the connecting elements; the auxiliary section is connected to the arc-shaped section to restrain the heart valve in the human body; the anchoring part can be connected with the connecting element, and the anchoring part is arranged around the periphery of the valve stent after it is connected with the connecting element, and the auxiliary section is arranged near the outflow end and near the side of the body section with respect to the arc-shaped section.

Preferably, in any of the above-mentioned interventional artificial heart valves or any of the above-mentioned medical apparatuses, the body section has an inward retracted area near the outflow end.

Preferably, in any of the above-mentioned interventional artificial heart valves or any of the above-mentioned medical apparatuses, an internal sealing film is arranged inside the valve stent.

Preferably, in any of the above-mentioned interventional artificial heart valves or any of the above-mentioned apparatuses, the skirt section has a trumpet-shaped structure, the body section is generally cylindrical, and a smaller diameter end of the trumpet-shaped structure of the skirt section is connected to the body section;
the peripheral edge of the trumpet-shaped structure has a downward flanged structure for enhancing an effect of anchoring to a native valve annulus.

Preferably, in any of the above-mentioned interventional artificial heart valves or any of the above-mentioned medical apparatuses, the cross section of the body section and/or the skirt section is a D-shaped or elliptical closed ring enclosed by a plurality of convex curves.

The above technical solution employed by the present invention can attain the following beneficial effects:
In the present invention, on the basis of employing a non-closed annular anchoring part, the structure of the valve stent and the connecting structure of the valve stent for connecting with the anchoring part are improved, so that the interventional artificial heart valve is suitable for being implanted into the left atrium or the right atrium of the human heart through a catheter to replace a diseased mitral valve or tricuspid valve to work. After the implantation, it will not cause damage to the normal physiological function of the human valve leaflets, and has a certain restriction effect on the expansion of the valve annulus tissue, and the outflow tract obstruction can be reduced, and the postoperative rehabilitation of the patient can be promoted. Besides, the flexible annular seal uses a material capable of absorbing blood. After the artificial heart valve is implanted, the flexible annular seal absorbs blood, gradually expands, swells and thickens, thereby fully fills the clearance between the valve stent and skirt section, the human heart valve and the human valve annulus, gives proper compression to the human tissues, thus avoids or reduces the occurrence of perivalvular leakage. The auxiliary section can work with the bands of supporting part to attain a synergistic effect to ensure the stability of the anchoring elements, and further lift the human heart valve at the same time, thus reduces outflow tract obstruction.

### Brief Description of Drawings

In order to explain the technical solution of the examples of the present invention more clearly, hereunder the accompanying drawings, which will be used in the description of the examples and constitute a part of the present invention, will be described briefly. The exemplary examples of the present invention and associated description are intended to explain the present invention, but don't constitute any undue limitation to the present invention. In the figures:
FIG. 1 is a schematic structural diagram of the valve stent of an interventional artificial heart valve disclosed in example 1 of the present invention;
FIG. 2 is a schematic diagram of the combined structure of the interventional artificial heart valve disclosed in example 1 of the present invention;
FIG. 3 is a schematic plan view of the valve stent of the interventional artificial heart valve disclosed in example 1 of the present invention;
FIG. 4A is a schematic plan view of the valve stent of the interventional artificial heart valve disclosed in example 1 of the present invention;
FIG. 4B is a schematic plan view of another embodiment of the valve stent of the interventional artificial heart valve disclosed in example 1 of the present invention;
FIG. 5 is a top view of the valve stent of the interventional artificial heart valve disclosed in example 1 of the present invention;
FIG. 6 is a schematic diagram of the combined structure of the interventional artificial heart valve disclosed in example 2 of the present invention;
FIG. 7 is a schematic diagram of the combined structure of the interventional artificial heart valve disclosed in example 2 of the present invention;
FIG. 8 is a schematic diagram of the combined structure of the interventional artificial heart valve disclosed in example 3 of the present invention;
FIG. 9 is a schematic diagram of the combined structure of the interventional artificial heart valve disclosed in example 3 of the present invention;
FIG. 10 is a schematic diagram of the combined structure of the interventional artificial heart valve disclosed in example 3 of the present invention;
FIG. 11 is a schematic diagram of the combined structure of the interventional artificial heart valve disclosed in example 4 of the present invention;
FIG. 12A and FIG. 12B are schematic diagrams of the combined structure of the interventional artificial heart valve disclosed in example 5 of the present invention;
FIG. 13 is a schematic diagram of the state of the interventional artificial heart valve disclosed in example 6 of the present invention during implantation;
FIG. 14 is a schematic diagram of the interventional artificial heart valve disclosed in example 6 of the present invention after it is implanted into the heart;
FIG. 15 is a schematic diagram of the interventional artificial heart valve disclosed in example 6 of the present invention after it is implanted into the heart.

Description of the reference numerals:
100 - ventricle; 200 - human heart valve; 300 - human valve annulus; 400 - chordae tendinea of heart; 500 - interventional artificial heart valve;
1 - valve stent; 10 - body section; 11 - supporting part; 111 - first band of supporting part; 112 - second band of supporting part; 1111, 1121 - convergent part; 12 - restraining part; 120 - skirt section; 121 - downward flanged structure; 13 - valve leaflet part; 14 - inward retracted area; 15 - flexible annular seal; 16 - first connecting element; 17 - second connecting element; 161 - first connecting column, 162 - second connecting column, 171 - third connecting column, 172 - fourth connecting column; 2 - anchoring part; 21 - first anchoring element; 22 - second anchoring element; 211 - first socket; 212 - second socket; 221 - third socket; 222 - fourth socket; 23 - arc-shaped section; 24 - anti-loose structure; 25 - auxiliary section; 26 - connecting section; 30 - guide wire.

### Embodiments

To make the object, technical solution, and advantages of the present invention understood more clearly, hereunder the technical solution of the present invention will be detailed clearly and completely in embodiments, with reference to the accompanying drawings of the present invention. In the description of the present invention, it should be noted that the term "or" is usually used with the meaning of including "and/or", unless otherwise indicated explicitly in the context.

It should be noted: in the description of the present invention, unless otherwise specified and defined explicitly, the terms "install", "interconnect", and "connect" shall be interpreted in their general meanings, for example, a connection may be a fixed connection, a detachable connection, or an integral connection; may be a mechanical connection or an electrical connection; or may be a direct connection or an indirect connection via an intermediate medium, or internal communication between two elements. Those having ordinary skills in the art can understand the specific meanings of the terms in the present invention according to specific situations.

As described herein, the "outflow tract" refers to the left ventricular outflow tract in the case that the interventional artificial heart valve 500 is a mitral valve, and refers to the right ventricular outflow tract in the case that the interventional artificial heart valve 500 is a tricuspid valve.

As described herein, "generally enclosed" refers to an open space enclosed by open curved surfaces rather than a closed space enclosed by complete curved surfaces. "Generally annular" may refer to a generally annular shape combined from a plurality of components, which is not limited to a closed annular shape or a standard annular shape.

Apparently, the examples described herein are only a part of examples of the invention rather than all examples of the present invention. Other examples obtained by those having ordinary skills in the art on the basis of the examples described herein without expending any creative labor shall be deemed as falling in the scope of protection of the present invention.

### Example 1

An interventional artificial heart valve 500 is provided in this example 1, mainly comprising an anchoring part 2 in a substantially annular shape arranged in the vicinity of the native valve annulus tissue and a valve stent 1 provided with artificial leaflets.

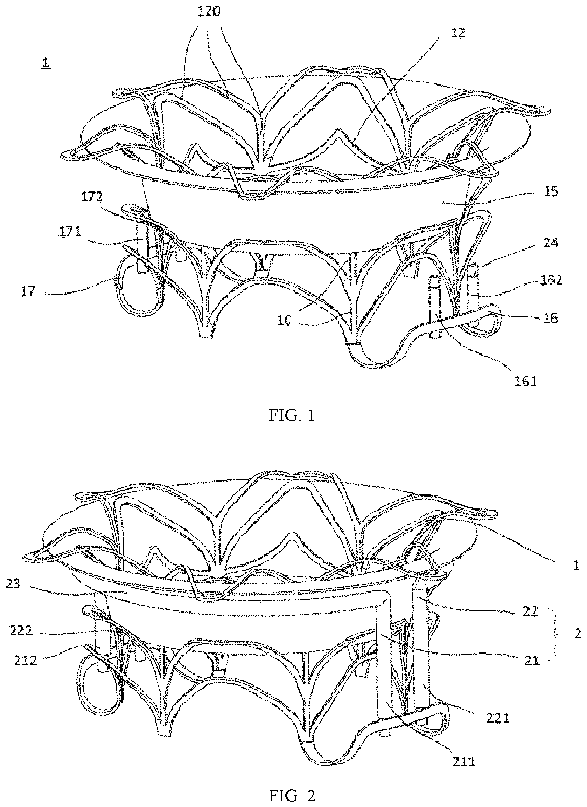

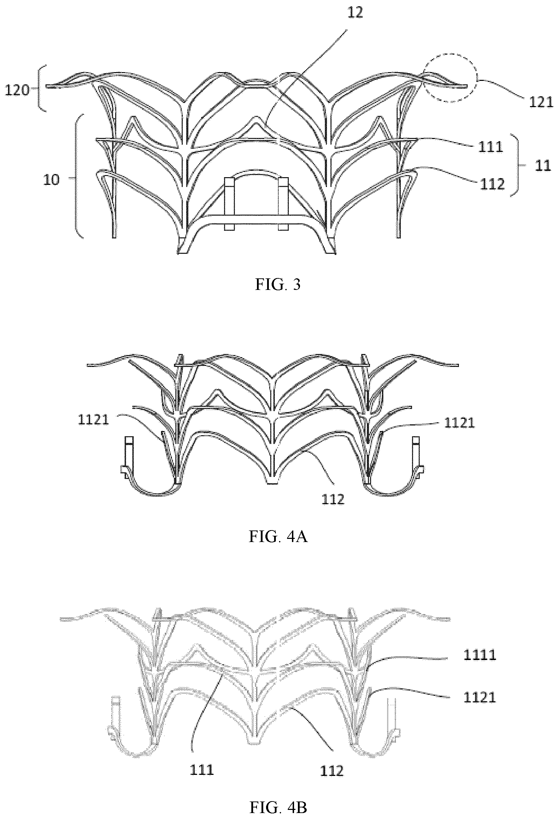

As shown in FIGS. 1 and 3, an atrial flange for joining to a human valve annulus 300 is provided at a top inflow end of the valve stent 1; specifically, the valve stent 1 has a body section 10 and a skirt section 120 arranged at the inflow end; preferably, the skirt section 120 has a trumpet-shaped structure, the body section 10 is generally cylindrical, and a minimum diameter end of the trumpet-shaped structure of the skirt section 120 is connected to the body section 10. Preferably, the body section 10 comprises a longitudinal skeleton and a transverse skeleton that are interconnected or interlaced with each other. In a preferred example, the transverse skeleton can be replaced by other structures, so as to provide better radial collapse performance while achieving other functions, as discussed in detail below.

As shown in FIG. 4A, a valve leaflet part 13 is arranged inside the body section 10; the valve leaflet part 13 preferably has two or three artificial valve leaflets attached to the struts of the valve stent 1, and an internal sealing film is arranged inside the valve stent 1 to prevent perivalvular leakage.

In view that the interventional artificial heart valve 500 in the example 1 is to be delivered through a catheter, the valve stent 1 can radially collapse and expand between a radially collapsed configuration and a radially expanded configuration.

At least two connecting elements are arranged at a side of an outflow end of the body section 10; the lower end of the valve stent 1 is provided with a corresponding number of connecting parts protruding radially outward for receiving the connecting elements on the annular anchoring elements.

As shown in FIG. 2, the anchoring part 2 is arranged around the periphery of the valve stent 1 and detachably connected to the connecting elements; and the anchoring part 2 comprises at least two anchoring elements, that is to say, the anchoring part 2 has at least two separate sections. After a plurality of sections, in other words, a plurality of anchoring elements, are correspondingly connected with the connecting elements, the anchoring elements can generally enclose to form an annular shape. Those skilled in the art should understand that not all clearance cannot exist among the anchoring elements, clearance may exist among the anchoring elements, small clearance doesn't affect the effect of the example 1 and doesn't lead to problems such as regurgitation. In the process of implementation, the annular anchoring part 2 is placed in the ventricle 100, below the human valve annulus 300, at the periphery of the human heart valve 200, and the anchoring part 2 and the valve stent 1 form interference fit between them, so as to effectively keep the interventional artificial heart valve 500 in an ideal implantation position. By dividing the annular anchoring part 2 into a plurality of sections (anchoring elements), the annular anchoring elements can be move around the chordae tendineae of heart 400 in an open configuration and arranged on the back of the valve leaflet tissue.

At least a part of the outer surface of the skirt section 120 is covered by a flexible annular seal 15, the flexible annular seal 15 comprises a material capable of absorbing blood; preferably, the flexible annular seal 15 further covers a partial area of the body section 10 at the side of the inflow end.

In the example 1, the material capable of absorbing blood is one or more of PET fibers, polyester fibers and polyamide fibers. Since the flexible annular seal 15 uses a material capable of absorbing blood, during the interventional operation, the flexible annular seal 15 covers a partial area of the human heart valve 200 and a partial area of the human valve annulus 300 when it enters the human heart through a catheter. When the flexible annular seal 15 comes into contact with the blood, the above-mentioned material capable of absorbing blood will gradually absorb the blood, and will undergo morphological changes such as expansion, swelling and thickening within several minutes to tens of minutes. That is to say, after the anchoring part 2 and the valve stent 1 are connected in place, the flexible annular seal 15 gradually expands, swells and thickens, fully fills the clearance among the valve stent 1, the skirt section 120, the human heart valve 200 and the human valve annulus 300, and gives proper pressure to the human tissues to avoid or reduce the occurrence of perivalvular leakage. In addition, since clamping force is applied to the valve annulus and/or the human heart valve 200 via the flexible annular seal 15, the pressure is applied uniformly, further avoiding or reducing the damage of the valve stent 1 to the human tissues.

In the example 1, the anchoring elements and the valve stent 1 are preferably made of a shape memory alloy, for example, preferably Ni-Ti alloy, so as to be delivered in a compressed form to the human heart valve 200 through a catheter, and then can expand to a predetermined form after being released by the catheter sheath. Preferably, the external surface of the annular anchoring element is covered by a film made of PET, PTFE or polyurethane, which reduces the damage to the captured leaflet tissue and is beneficial to promoting an endothelialization process.

As shown in FIG. 3, preferably, the body section 10 comprises a plurality of supporting parts 11 arranged circumferentially; the supporting parts 11 extend radially outward and towards the inflow end. Preferably, the supporting part 11 is generally a "herringbone" shaped structure, with two bottom ends connected to a vertical skeleton of the body section 10 respectively, and the top end of the structure is arc-shaped. Preferably, no horizontal skeleton is arranged on the body section 10; instead, a plurality of "herringbone" shaped supporting parts 11 evenly distributed in the circumferential direction of the body section 10 provide partial function of a horizontal skeleton to support the radial structure of the body section 10. The angle between the plane of the main body (i.e., the top and middle part of the "herringbone" shaped structure) of the supporting part 11 and the body section 10 at the joint is 30 to 60 degrees, preferably is 50 degrees. It should be understood that the plurality of supporting parts 11 and the skirt section 120 jointly define an internal channel for receiving the anchoring part 2 to some extent. Through the cooperation between the skirt section 120 and the supporting parts 11, the anchoring stability of the interventional artificial heart valve 500 is ensured, and the displacement after implantation is avoided to some extent.

As a preferred technical solution, the supporting part 11 comprises a first band of supporting part 111 and a second band of supporting part 112 that are arranged in the circumferential direction, wherein the first band of supporting part 111 is closer to the side of the inflow end than the second band of supporting part 112. Preferably, the first band of supporting part 111 and the second band of supporting part 112 have a structure of 6 supporting parts 11 respectively. The second band of supporting part 112 is near the lower outflow end of the valve stent 1, and provides a function of valve leaflet clamping to some extent, for folding and clamping the valve leaflet tissue. During the execution of the interventional surgery, the leaflet tissue is captured and held therein by the first band of supporting part 111 and the second band of supporting part 112. By lifting the leaflet tissue upward near the outflow tract of the ventricle 100, the effective outflow area near the outflow tract is increased, and the effect of reducing outflow tract obstruction is achieved.

As a preferred example, as shown in FIG. 4A, the second band of supporting part 112 has convergent parts 1121 near the first connecting element 16 and the second connecting element 17. Specifically, a supporting part 11 of the second band of supporting part 112 closest to the first connecting element 16 and another supporting part 11 of the second band of supporting part 112 closest to the second connecting element 17 are two convergent parts 1121 respectively. The supporting part 11 of the convergent part 1121 is more convergent radially than the other supporting parts 11 of the second band of supporting part 112. Preferably, the convergent part 1121 extends toward the inflow end rather than extend radially outward.

As another preferred example, as shown in FIG. 4B, the first band of supporting part 111 and the second band of supporting part 112 have convergent parts 1111 and convergent parts 1121 near the first connecting element 16 and the second connecting element 17. Specifically, a supporting part 11 of the first band of supporting part 111 closest to the first connecting element 16 and another supporting part 11 of the first band of supporting part 111 closest to the second connecting element 17 are two convergent parts 1111 respectively; a supporting part 11 of the second band of supporting part 112 closest to the first connecting element 16 and another supporting part 11 of the second band of supporting part 112 closest to the second connecting element 17 are two convergent parts 1121 respectively. The supporting part 11 of the convergent part 1121 is more convergent radially than the other supporting parts 11 of the second band of supporting part 112. Preferably, the convergent parts 1121 extend toward the inflow end rather than extend radially outward. This example can avoid the interference between the anchoring part 2 and the valve stent 1 to the greatest extent, and prevent the anchoring part 2 from hooking on the supporting part 11 of the valve stent 1 during the operation, thus reducing the difficulty of operation.

On that basis, as a preferred example, as shown in FIG. 4B, compared with the example shown in FIG. 4A, the first connecting element 16 and the second connecting element 17 have smaller radial outward extension, so that the anchoring part 2 can enhance the anchoring effect on the human heart valve 200.

As shown in FIG. 3, since the flexible annular seal 15 expands after absorbing blood, the expanded part may have an unexpected excessive expansion in the uncovered area of the valve stent 1, and intrude into the valve stent 1 excessively over time, resulting in a narrowed interior of the valve stent 1. Therefore, as a preferred technical solution, the body section 10 comprises a restraining part 12, which is arranged at an edge of the body section 10 at the inflow end to restrain the flexible annular seal 15 from intruding into the internal space of the valve stent 1. The restraining part 12 comprises a plurality of lateral restraining frames, which are generally in a "herringbone" shaped structure respectively. The two bottom ends of the structure are connected to the top of the inflow end of the vertical skeleton of the body section 10 respectively, and the top end of the structure is in a circular arc shape. The "herringbone" shaped structure of the lateral restraining frame can optimize the folding performance of the valve stent 1.

As shown in FIG. 2, as a preferred technical solution, the anchoring element has an arc-shaped section 23, and a connecting part is arranged at a free end of the arc-shaped section 23. Two corresponding free ends of each section are provided with connecting elements for connecting to the valve stent 1.

Preferably, both the arc-shaped section 23 and the connecting part of the anchoring element are hollow tubular elements.

Preferably, the arc-shaped section 23 has a greater diameter than the connecting part, so as to increase the contact area between the anchoring element and the human tissues, and decrease the risk of damage to the human tissues while increasing the anchoring stability.

Preferably, as shown in FIGS. 1-2, the first connecting element 16 and the second connecting element 17 are provided at the edge of the body section 10 at the side of the outflow end, the first connecting element 16 is provided with a first connecting column 161 and a second connecting column 162, and the second connecting element 17 is provided with a third connecting column 171 and a fourth connecting column 172; the anchoring part 2 comprises a first anchoring element 21 and a second anchoring element 22, a connecting part of the first anchoring element 21 comprises a first socket 211 and a second socket 212, and a connecting part of the second anchoring element 22 comprises a third socket 221 and a fourth socket 222; and the first socket 211, the second socket 212, the third socket 221 and the fourth socket 222 can receive the first connecting column 161, the third connecting column 171, the second connecting column 162 and the fourth connecting column 172 respectively.

Furthermore, preferably, the first socket 211, the second socket 212, the third socket 221 and the fourth socket 222 and/or the first connecting column 161, the second connecting column 162, the third connecting column 171 and the fourth connecting column 172 have an anti-loose structure 24 respectively.

Preferably, the anti-loose structure 24 comprises one or more of pawl, ratchet, groove, flange and snap so as to ensure the stability of the connection. Those skilled in the art should understand that the anti-loose structure 24 can be selected from any similar anti-loose structure 24 existing in the art, and there is no particular restriction on the anti-loose structure in the present application. Preferably, all of the first connecting column 161, the second connecting column 162, the third connecting column 171 and the fourth connecting column 172 are hollow columns.

As shown in FIG. 3, the peripheral edge of the trumpet-shaped structure of the skirt section 120 has a downward flanged structure 121 for enhancing an effect of anchoring to a native valve annulus. The downward flanged structure 121 can cooperate with the anchoring part 2 to create a clamping effect, so as to further improve the anchoring stability. Specifically, the trumpet-shaped structure of the skirt section 120 comprises six petaloid skeletons, each petaloid skeleton comprises a first section extending outward and upward from the top of the body section 10, and a second section extending outward and downward. It should be understood that the downward flanged structure 121 corresponds to the above-mentioned second section of the petaloid skeleton. Besides, the petaloid skeleton is gradually tapered as it extends outward. In this example, "outward" means a direction from the center of the trumpet-shaped structure toward the exterior, "upward" means a direction toward the inflow end, and "downward" means a direction toward the outflow end.

In addition, as a preferred technical solution, the cross section of the body section 10 and/or the skirt section 120 is a D-shaped or elliptical closed ring enclosed by a plurality of convex curves, so as to fit the structure of the human valve annulus 300 better.

In the interventional artificial heart valve 500 in the example 1, on the basis of employing a non-closed annular anchoring part 2, the structure of the valve stent 1 and the connecting structure of the valve stent 1 for connecting with the anchoring part 2 are improved, so that the interventional artificial heart valve 500 is suitable for being implanted into the left atrium or the right atrium of the human heart through a catheter to replace a diseased mitral valve or tricuspid valve. After the implantation, it will not cause damage to the normal physiological function of the human valve leaflets, and has a certain restriction effect on the expansion of the valve annulus tissue. The outflow tract obstruction can be reduced, and the postoperative rehabilitation of the patient can be promoted.

### Example 2

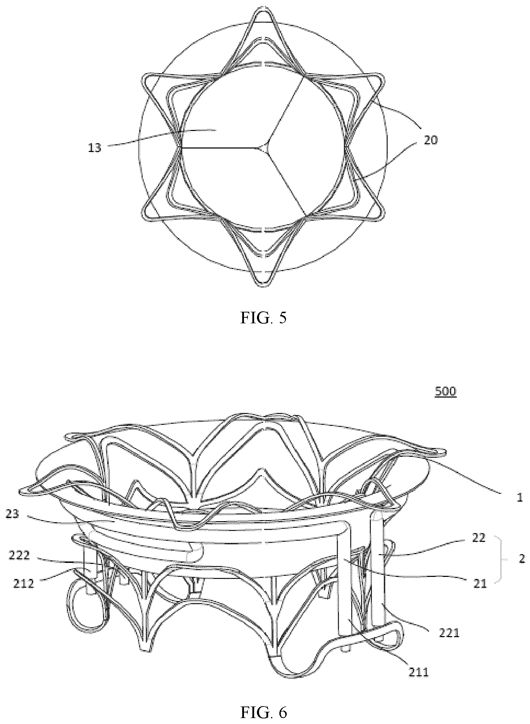

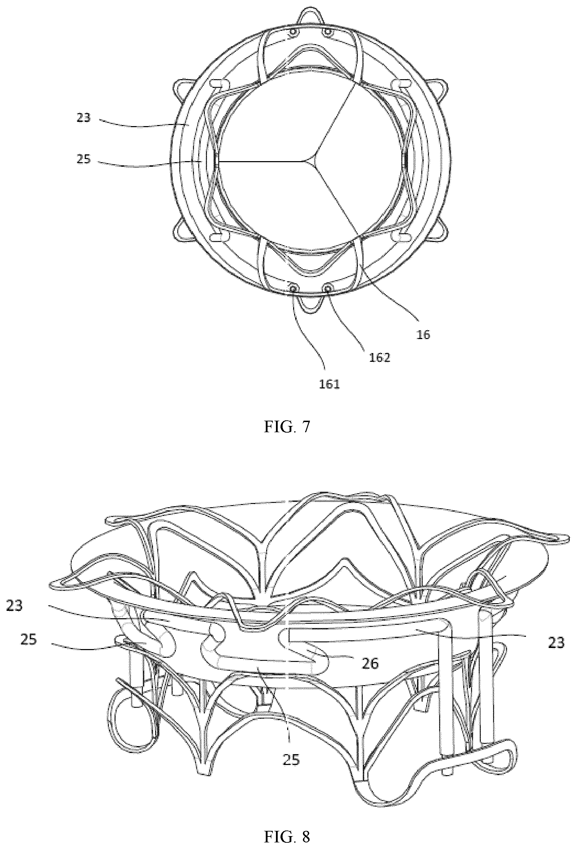

An interventional artificial heart valve 500 is provided in the example 2. The specific structure of the anchoring part 2 in the example 2 is different from that in the example 1, as shown in FIGS. 6 and 7. As a preferred technical solution, the anchoring element comprises an arc-shaped section 23, an auxiliary section 25 and a connecting part; the auxiliary section 25 is connected to the arc-shaped section 23. The connecting part is arranged at a free end of the arc-shaped section 23, and the connecting part can be detachably connected to the connecting elements.

The auxiliary section 25 is also in a circular arc shape, and the size of the circular arc of the auxiliary section 25 is smaller than that of the arc-shaped section 23. The auxiliary section 25 is connected with the arc-shaped section 23 by two connecting sections 26 at the two ends; all of the arc-shaped section 23, the auxiliary section 25, and the connecting sections 26 are located in an area generally enclosed by the first band of supporting part 111.

Preferably, the arc-shaped section 23 has a greater diameter than the connecting part; preferably the diameter of the arc-shaped section 23 is 1.5 to 2.5 times that of the connecting part, so as to increase the contact area between the anchoring element and the human tissues, and decrease the risk of damage to the human tissues while increasing the anchoring stability.

As shown in FIGS. 6 and 7, the arc-shaped section 23 is located in an area that is generally enclosed by the first band of supporting part 111; and the auxiliary section 25 is also located in the area that is generally enclosed by the first band of supporting part 111. The auxiliary section 25 comprises an arc-shaped auxiliary section and connecting sections 26 arranged at two ends of the auxiliary section, and the connecting sections 26 are connected with the arc-shaped section 23.

Preferably, the arc-shaped section 23 and the connecting parts at the two ends are hollow tubular, in which a communicated and smooth cavity is formed, allowing a guide wire 30 to pass through. The auxiliary section 25 is preferably hollow tubular or solid cylindrical; in addition, even if the auxiliary section 25 is hollow tubular, its inner cavity is not in communication with the inner cavity of the arc-shaped section 23, and doesn't allow a guide wire 30 to pass through.

Preferably, the arc-shaped section 23 has a greater diameter than the auxiliary section 25, and the diameter of the arc-shaped section 23 is preferably 1.5 to 2.5 times that of the auxiliary section 25. Besides, the arc-shaped section 23 has higher structural strength than that of the auxiliary section 25.

In the example 2, the auxiliary section 25 can restrain (press) the human heart valve 200 in the first band of supporting part 111 closer to the inflow end, so that the human heart valve 200 can be further lifted and the outflow tract obstruction can be further reduced. Besides, the auxiliary section 25 further works with the first band of supporting part 111 to achieve a synergistic effect. In the case that the arc-shaped section 23 can't tightly press the valve annulus owing to deformation, aging or other reasons, the auxiliary section 25 can provide supporting force to the arc-shaped section 23 to a certain extent, so as to ensure the stability of the anchoring element.

### Example 3

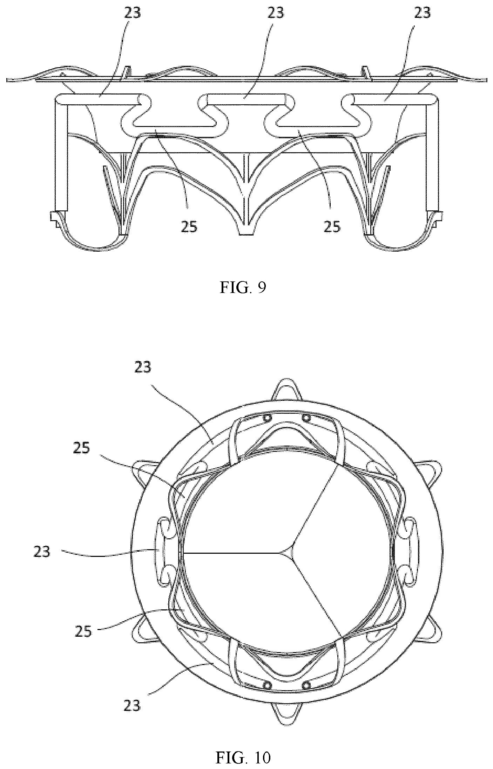

As shown in FIGS. 8-10, the specific structure of the anchoring part 2 in the example 3 is different from that in the example 2, while the valve stent 1 in this example is the same as that in the examples 1 and 2. The anchoring element has a generally arc-shaped section 23, the free end of the arc-shaped section 23 is provided with a connecting part. Two corresponding free ends of each section are provided with connecting elements for connecting to the valve stent 1. Both the arc-shaped section 23 and the connecting part of the anchoring element are hollow tubular elements. The arc-shaped section 23 has a greater diameter than the connecting part; preferably the diameter of the arc-shaped section 23 is 1.5 to 2.5 times that of the connecting part, so as to increase the contact area between the anchoring element and the human tissues, and decrease the risk of damage to the human tissues while increasing the anchoring stability.

As shown in FIGS. 8-10, the arc-shaped section 23 comprises a plurality of separate sections, and the auxiliary section 25 comprises a plurality of separate sections; the separate sections of the arc-shaped section 23 and the separate sections of the auxiliary section 25 are connected as a whole by a plurality of connecting sections 26; the arc-shaped section 23, the auxiliary section 25, and the connecting sections 26 are hollow tubular elements; and the auxiliary section 25 and the connecting sections 26 are also located in the area that is generally enclosed by the first band of supporting part 111.

Preferably, the arc-shaped section 23, the auxiliary section 25, the connecting section 26, and the connecting parts at the two ends are hollow tubular, in which a communicated and smooth cavity is formed, allowing a guide wire 30 to pass through. Preferably, the arc-shaped section 23 has the same diameter as the auxiliary section 25, and the arc-shaped section 23 and the auxiliary section 25 are preferably made of the same material integrally.

Preferably, the auxiliary section 25 comprises two separate sections, and the positions of the two sections correspond to the positions of the two supporting parts 11 of the first band of supporting part 111 in a state where the valve stent 1 is connected with the anchoring part 2, so as to produce a synergistic effect. The connecting section 26 is preferably not perpendicular to the arc-shaped section 23 and the auxiliary section 25, and the sum of the included angle of the connecting section 26 with the arc-shaped section 23 and the included angle with the auxiliary section 25 is 180 degrees. The length of the separate sections of the auxiliary section 25 is greater than the length of the gap between the sections of the arc-shaped section 23.

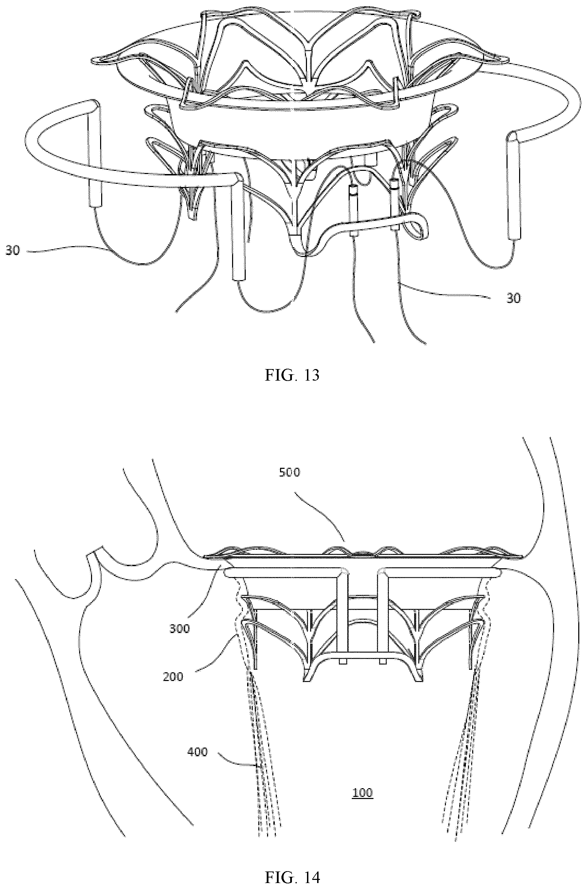

In the example 3, according to the effect illustrated in FIG. 14, the auxiliary section 25 can restrain (press) the human heart valve 200 in the first band of supporting part 111 closer to the inflow end, so that the human heart valve 200 can be further lifted and the outflow tract obstruction can be further reduced; besides, the auxiliary section 25 can work with the first band of supporting part 111 to achieve a synergetic effect. Moreover, owing to the integral structure of the anchoring element, the anchoring element is easier to manufacture and can provide better reliability.

### Example 4

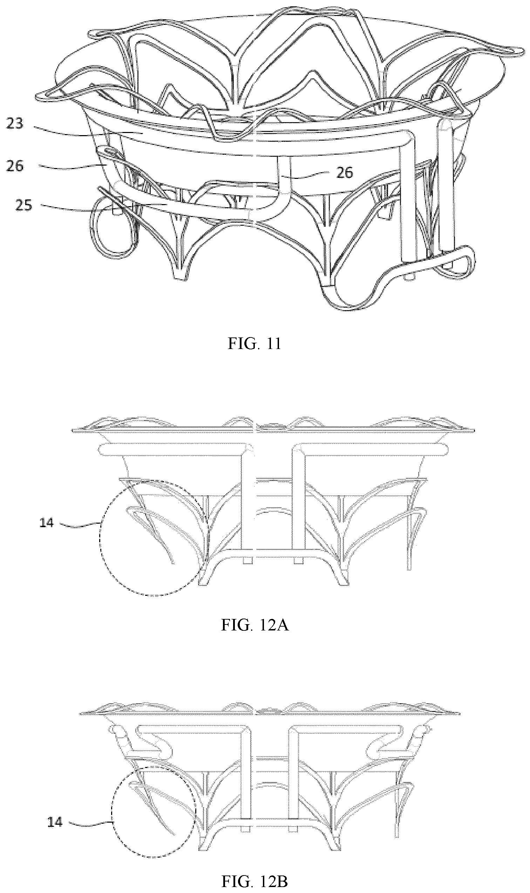

The specific structure of the anchoring part 2 in the example 4 is different from that in the examples 1 and 2. As shown in FIG. 11, the anchoring element comprises an arc-shaped section 23, an auxiliary section 25 and a connecting part; the auxiliary section 25 is connected to the arc-shaped section 23. The connecting part is provided at a free end of the arc-shaped section 23, and the connecting part can be detachably connected to the connecting elements.

The auxiliary section 25 is also in a circular arc shape, and the size of the circular arc is smaller than that of the arc-shaped section 23. The auxiliary section 25 is connected with the arc-shaped section 23 by two connecting sections 26 at the two ends; as shown in FIG. 11, the arc-shaped section 23 is located in an area generally enclosed by the first band of supporting part 111, while the auxiliary section 25 is located in an area generally enclosed by the second band of supporting part 112.

Preferably, the arc-shaped section 23 has a greater diameter than the connecting part; preferably the diameter of the arc-shaped section 23 is 1.5 to 2.5 times that of the connecting part, so as to increase the contact area between the anchoring element and the human tissues, and decrease the risk of damage to the human tissues while increasing the anchoring stability.

As shown in FIG. 11, the auxiliary section 25 is in a circular arc shape, and is connected to the arc-shaped section 23 by the connecting sections 26 at the two ends. Compared with the example 2, the connecting sections 26 in the example 4 are longer, and the two ends of each connecting section 26 span the first band of supporting part 111 and the second band of supporting part 112. Furthermore, the auxiliary section 25 and the connecting sections 26 connected at the two ends are connected to the arc-shaped section 23 across the two supporting parts 11 of the first band of supporting part 111.

Preferably, the arc-shaped section 23 and the connecting parts at the two ends are hollow tubular, in which a communicated and smooth cavity is formed, allowing a guide wire 30 to pass through. The auxiliary section 25 is preferably hollow tubular or solid cylindrical; in addition, even if the auxiliary section 25 is hollow tubular, its inner cavity is not in communication with the inner cavity of the arc-shaped section 23, and doesn't allow a guide wire 30 to pass through.

Preferably, the arc-shaped section 23 has a greater diameter than the auxiliary section 25, and the diameter of the arc-shaped section 23 is preferably 1.5 to 2.5 times that of the auxiliary section 25. Besides, the arc-shaped section 23 has higher structural strength than that of the auxiliary section 25.

In the example 4, the auxiliary section 25 can restrain (press) the human heart valve 200 in the second band of supporting part 112 closer to the inflow end, so that the human heart valve 200 can be further lifted and the outflow tract obstruction can be further reduced. Besides, since the second band of supporting part 112 is closer to the chordae tendineae 400, the anchoring structure can provide a certain restraining effect on the free end of the human heart valve 200 and the chordae tendineae 400, thus minimizes the outflow tract obstruction.

In a preferred example, the interventional artificial heart valve 500 has a first anchoring element 21 and a second anchoring element 22, wherein the first anchoring element 21 has the anchoring element structure in example 4 and is connected at the side near the inflow end of the aortic valve; the second anchoring element 22 has the anchoring element structure in example 2 or 3, and is connected at the other side away from the aortic valve. This example can reduce the outflow tract obstruction and provide a stable anchoring function at the same time.

### Example 5

An interventional artificial heart valve 500 is provided in this example 5. Different from the examples 1-4, as shown in FIGS. 12A and 12B, the entire valve stent 1 is designed asymmetrically, and a part of the outflow end of the body section 10 corresponding to the outflow tract of the ventricle 100 is offset away from the outflow tract, that is to say, the body section 10 has an inward retracted area 14 near the outflow end, so as to further reduce the influence on the outflow tract. Those skilled in that art should understand that the preferred position of the inward retracted area 14 should be away from the positions of the first connecting element 16 and the second connecting element 17. Preferably, the inward retracted area 14 is arranged at the same distance from the first connecting element 16 and the second connecting element 17 to minimize the influence on the outflow tract.

Furthermore, since the entire valve stent 1 is designed asymmetrically, the first anchoring element 21 and the second anchoring element 22 should be configured as different structures accordingly, and the anchoring element (taking the first anchoring element 21 as an example) at the side of the inward retracted area 14 has a smaller size. Specifically, the arc-shaped section 23 of the first anchoring element 21 is shorter than the arc-shaped section 23 of the second anchoring element 22, while it is unnecessary to change the shapes and sizes of the first socket 211, the second socket 212, the third socket 221 and the fourth socket 222.

### Example 6

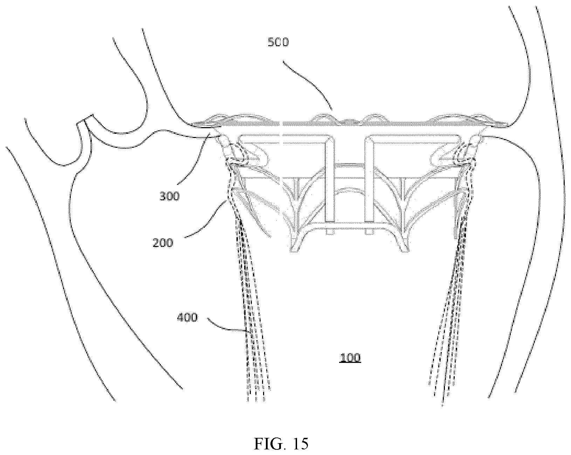

In the example 6, the implantation process of the interventional artificial heart valve 500 in the examples 1-5 will be described with reference to FIGS. 13-15.

In the process of implantation, first, a plurality of guide wires 30 are arranged at the lower part of the tissue of the human valve annulus 300, and the first anchoring element 21 and the second anchoring element 22 of the anchoring part 2 are delivered to the implantation site along the pre-arranged guide wires 30 by means of a first delivery device, and then are released at the implantation site; and each guide wire 30 is threaded through the connecting elements at the two ends of the first anchoring element 21 and the second anchoring element 22, respectively.

Then, the guide wires 30 are threaded through the corresponding connecting parts on the valve stent 1, such as the first connecting column 161 of the first connecting element 16, the third connecting column 171 of the second connecting element 17, the second connecting column 162 of the first connecting element 16 and the fourth connecting column 172 of the second connecting element 17, and then the valve stent 1 in a compressed state is delivered into the heart by means of a second delivery device.

The guide wires 30 are used to connect the connecting elements on the annular anchoring elements and the corresponding connecting parts. For example, the first socket 211, the second socket 212, the third socket 221 and the fourth socket 222 receive the first connecting column 161, the third connecting column 171, the second connecting column 162 and the fourth connecting column 172 respectively, and are secured by means of the anti-loose structures 24.

Next, by withdrawing the protective sheath covering the outside of the valve stent 1, the valve stent 1 expands to capture the native valve leaflets and tightly fit with the annular anchoring elements, thus the implantation procedure is completed.

Then, the flexible annular seal 15 is placed to cover a partial area of the human heart valve 200 and a partial area of the valve annulus. When the flexible annular seal 15 comes into contact with the blood, the material capable of absorbing blood gradually absorb the blood, and the flexible annular seal 15 undergoes morphological changes such as expansion, swelling and thickening within several minutes to tens of minutes after the above-mentioned implantation process is completed. Finally, the flexible annular seal 15 gradually expands, swells and thickens, fully fills the clearance among the valve stent 1, the skirt section 120, the human heart valve 200 and the human valve annulus 300, and applies proper pressure on the human tissues to further avoid or mitigate the occurrence of perivalvular leakage.

In the example 6, as an example, the implantation process of the interventional artificial heart valve 500 in the examples 2-4 will be further described.

In the process of implantation, first, a plurality of guide wires 30 are arranged at the lower part of the tissue of the human valve annulus 300, and the first anchoring element 21 and the second anchoring element 22 of the anchoring part 2 are delivered to the implantation site along the pre-arranged guide wires 30 by means of a first delivery device, and then are released at the implantation site; and each guide wire 30 is threaded through the connecting elements at the two ends of the first anchoring element 21 and the second anchoring element 22, respectively.

Wherein, the process of threading the guide wires 30 through the first anchoring element 21 and the second anchoring element 22 may vary depending on the specific structure of the anchoring elements. For the first anchoring element 21 and the second anchoring element 22 in the examples 2 and 4, the guide wires 30 only have to be threaded through the hollow tubular body of the arc-shaped section 23.

For the technical solution in the example 3, the arc-shaped section comprises a plurality of separate sections, and the auxiliary section 25 also comprises a plurality of separate sections; the separate sections of the arc-shaped section and the separate sections of the auxiliary section 25 are connected as a whole by a plurality of connecting sections; all of the arc-shaped section, the auxiliary section 25 and the connecting sections are hollow tubular elements. It should be understood that the guide wires 30 must be threaded through the cavities of all sections of the first anchoring element 21 and the second anchoring element 22. In the guide wire threading process, preferably the first anchoring element 21 and the second anchoring element 22 are tensioned to reduce their bending and make their internal cavities as smooth as possible to facilitate threading.

Then, the guide wires 30 are threaded through the corresponding connecting parts on the valve stent 1, such as the first connecting column 161 of the first connecting element 16, the third connecting column 171 of the second connecting element 17, the second connecting column 162 of the first connecting element 16 and the fourth connecting column 172 of the second connecting element 17, and then the valve stent 1 in a compressed state is delivered into the heart by means of a second delivery device.

The guide wires 30 are used to connect the connecting elements on the annular anchoring elements and the corresponding connecting parts. For example, the first socket 211, the second socket 212, the third socket 221 and the fourth socket 222 receive the first connecting column 161, the third connecting column 171, the second connecting column 162 and the fourth connecting column 172 respectively, and are secured by means of the anti-loose structures 24.

Next, by withdrawing the protective sheath covering the outside of the valve stent 1, the valve stent 1 expands to capture the native valve leaflets and tightly fit with the annular anchoring elements, thus the implantation procedure is completed.

### Example 7

A medical apparatus for improving the function of a heart valve is provided in the example 7. The medical apparatus may be understood as a medical kit for improving the function of a heart valve. The medical apparatus or medical kit mainly comprises two parts, i.e., a valve stent 1 and an anchoring part 2. In addition, the apparatus or kit may further include a catheter and/or guide wires 30.

The valve stent 1 has a body section 10 and a skirt section 120 at an inflow end; a valve leaflet part 13 is arranged in the body section 10; the valve stent 1 can radially collapse and expand between a radially collapsed configuration and a radially expanded configuration; at least a part of the outer surface of the skirt section 120 is covered by a flexible annular seal 15, the flexible annular seal 15 comprises a material capable of absorbing blood; the material capable of absorbing blood expands after it absorbs blood; at least two connecting elements are arranged at a side of the outflow end of the body section 10; the valve stent 1 can be placed in a catheter in a radially collapsed state;

The anchoring part 2 comprises at least two anchoring elements 21 and 22, each of which comprises an arc-shaped section 23 and a connecting part provided at a free end of the arc-shaped section 23; the connecting part can be detachably connected to the connecting elements 16 and 17; and the anchoring part 2 has elasticity and can be placed in a catheter after elastic deformation.

In the medical apparatus or medical kit, both the valve stent 1 and the anchoring part 2 can be provided in a radially collapsed state or a radially expanded state before the interventional operation is performed.

The medical apparatus or medical kit may include an operating instruction, in which the implantation method and steps described in the above example 7 are recorded.

For the specific structure of the valve stent 1 and the anchoring part 2, please refer to all the features described in the examples 1-5 in this specification.

While some examples of the present invention are described above with reference to the accompanying drawings, the present invention is not limited to those examples. The examples described above are only illustrative rather than limiting. Various modifications and alternations may be made by those having ordinary skills in the art inspired by the present invention without departing from the scope of protection defined by the claims. However, all of such modifications and alternations shall be deemed as falling in the scope of protection of the present invention.

## Claims

1. An interventional artificial heart valve (500), comprising a valve stent (2) and an anchoring part, wherein the valve stent (2) has a body section (10) and a skirt section (120) at an inflow end; a valve leaflet part is arranged in the body section (10); the valve stent (2) can be radially collapsed and expanded between a radially collapsed configuration and a radially expanded configuration; at least a part of the outer surface of the skirt section (120) is covered by a flexible annular seal (15), the flexible annular seal (15) comprises a material capable of absorbing blood; the material capable of absorbing blood expands after it absorbs blood; at least two connecting elements (17) are arranged at a side of an outflow end of the body section (10); and the anchoring part is arranged around the periphery of the valve stent (2) and detachably connected to the connecting elements (17); the anchoring part comprises at least two anchoring elements, wherein the anchoring element has an arc-shaped section (23), an auxiliary section (25) and a connecting part, wherein the connecting part is provided at a free end of the arc-shaped section (23), and the connecting part can be detachably connected to the connecting elements (17); and the auxiliary section (25) is connected to the arc-shaped section (23) to restrain the heart valve in the human body; wherein the anchoring part can be connected with the connecting element, and is arranged around the periphery of the valve stent (2) after it is connected with the connecting element, and the auxiliary section (25) is arranged near the outflow end and near the side of the body section (10) with respect to the arc-shaped section (23); wherein the body section (10) comprises a plurality of supporting parts (11) arranged circumferentially; and the supporting parts (11) extend radially outward and towards the inflow end.

2. The interventional artificial heart valve according to claim 1, wherein the flexible annular seal further covers a partial area of the body section at the side of the inflow end.

3. The interventional artificial heart valve according to claim 1, wherein the body section comprises a restraining part, the restraining part is arranged at the edge of the body section at the side of the inflow end to restrain the flexible annular seal from intruding into an internal space inside the valve stent.

4. The interventional artificial heart valve according to claim 1, wherein the anchoring element has an arc-shaped section, a free end of the arc-shaped section is provided with a connecting part.

5. The interventional artificial heart valve according to claim 1, wherein the supporting part comprises a first band of supporting part and a second band of supporting part that are arranged in the circumferential direction, wherein the first band of supporting part is closer to the side of the inflow end than the second band of supporting part.

6. The interventional artificial heart valve according to claim 5, wherein
the arc-shaped section is located in an area that is generally enclosed by the first band of supporting part; and
the auxiliary section is located in an area that is generally enclosed by the first band of supporting part or the second band of supporting part.

7. The interventional artificial heart valve according to claim 6, wherein the auxiliary section comprises an arc-shaped auxiliary section and connecting sections arranged at two ends of the auxiliary section, and the connecting sections are connected with the arc-shaped section; and the auxiliary section is located in an area that is generally enclosed by the first band of supporting part.

8. The interventional artificial heart valve according to claim 6, wherein the arc-shaped section comprises a plurality of separate sections, and the auxiliary section comprises a plurality of separate sections; the separate sections of the arc-shaped section and the separate sections of the auxiliary section are connected together by a plurality of connecting sections; the arc-shaped section, the auxiliary section, and the connecting sections are hollow tubular elements; and the auxiliary section is located in an area that is generally enclosed by the first band of supporting part.

9. The interventional artificial heart valve according to claim 6, wherein the auxiliary section comprises an arc-shaped auxiliary section and connecting sections arranged at two ends of the auxiliary section, and the connecting sections are connected with the arc-shaped section; and the auxiliary section is located in an area that is generally enclosed by the second band of supporting part.

10. The interventional artificial heart valve according to claim 5, wherein the first band of supporting part and/or the second band of supporting part have a convergent part near each of the two connecting elements, and a supporting part of the convergent part is more convergent radially than other supporting parts of the first band of supporting part and the second band of supporting part.

11. The interventional artificial heart valve according to claim 1, wherein a first connecting element and a second connecting element are provided at the edge of the body section at the side of the outflow end, the first connecting element is provided with a first connecting column and a second connecting column, and the second connecting element is provided with a third connecting column and a fourth connecting column;
the anchoring part comprises a first anchoring element and a second anchoring element, a connecting part of the first anchoring element comprises a first socket and a second socket, and a connecting part of the second anchoring element comprises a third socket and a fourth socket; and
the first socket, the second socket, the third socket and the fourth socket can receive the first connecting column, the third connecting column, the second connecting column and the fourth connecting column respectively.

12. An interventional artificial heart valve according to claim 1, wherein the valve stent can be placed in a catheter in a radially collapsed state, and the anchoring part has elasticity and can be placed in a catheter after elastic deformation.

13. The interventional artificial heart valve according to any of claims 1-11 or the medical apparatus according to claim 12, wherein the body section has an inward retracted area near the outflow end.

14. The interventional artificial heart valve according to any of claims 1-11 or the medical apparatus according to claim 12, wherein the skirt section has a trumpet-shaped structure, the body section is generally cylindrical, and a smaller diameter end of the trumpet-shaped structure of the skirt section is connected to the body section;
the peripheral edge of the trumpet-shaped structure has a downward flanged structure for enhancing an effect of anchoring to a native valve annulus.

## Patentansprüche

1. Interventionelle künstliche Herzklappe (500), umfassend einen Klappenstent (2) und ein Verankerungsteil, wobei der Klappenstent (2) einen Körperabschnitt (10) und einen Schürzenabschnitt (120) an einem Einströmende aufweist; ein Klappensegelteil im Körperabschnitt (10) angeordnet ist; der Klappenstent (2) zwischen einer radial komprimierten Konfiguration und einer radial expandierten Konfiguration in radialer Richtung komprimiert und expandiert werden kann; mindestens ein Teil der Außenoberfläche des Schürzenabschnitts (120) von einer flexiblen ringförmigen Dichtung (15) bedeckt ist, wobei die flexible ringförmige Dichtung (15) ein blutabsorbierendes Material umfasst; das blutabsorbierende Material expandiert, nachdem es Blut absorbiert; mindestens zwei Verbindungselemente (17) an einer Seite eines Ausströmendes des Körperabschnitts (10) angeordnet sind; und das Verankerungsteil um den Umfang des Klappenstents (2) herum angeordnet und lösbar mit den Verbindungselementen (17) verbunden ist; das Verankerungsteil mindestens zwei Verankerungselemente umfasst, wobei das Verankerungselement einen bogenförmigen Abschnitt (23), einen Hilfsabschnitt (25) und einen Verbindungsabschnitt aufweist, wobei der Verbindungsabschnitt an einem freien Ende des bogenförmigen Abschnitts (23) vorgesehen ist, und der Verbindungsabschnitt lösbar mit den Verbindungselementen (17) verbunden werden kann; und der Hilfsabschnitt (25) mit dem bogenförmigen Abschnitt (23) verbunden ist, um die Herzklappe im menschlichen Körper zurückzuhalten; wobei das Verankerungsteil mit dem Verbindungselement verbunden werden kann, und nachdem es mit dem Verbindungselement verbunden ist, um den Umfang des Klappenstents (2) herum angeordnet ist, und der Hilfsabschnitt (25) nahe dem Ausströmende und nahe der Seite des Körperabschnitts (10) in Bezug auf den bogenförmigen Abschnitt (23) angeordnet ist; wobei der Körperabschnitt (10) eine Vielzahl von in Umfangsrichtung angeordneten Stützabschnitten (11) umfasst; und sich die Stützabschnitte (11) radial nach außen und zum Einströmende hin erstrecken.

2. Interventionelle künstliche Herzklappe nach Anspruch 1, wobei die flexible ringförmige Dichtung ferner einen Teilbereich des Körperabschnitts an der Seite des Einströmendes bedeckt.

3. Interventionelle künstliche Herzklappe nach Anspruch 1, wobei der Körperabschnitt einen Rückhalteabschnitt umfasst, wobei der Rückhalteabschnitt am Rand des Körperabschnitts an der Seite des Einströmendes angeordnet ist, um die flexible ringförmige Dichtung daran zu hindern, in einen inneren Raum innerhalb des Klappenstents einzudringen.

4. Interventionelle künstliche Herzklappe nach Anspruch 1, wobei das Verankerungselement einen bogenförmigen Abschnitt aufweist, wobei ein freies Ende des bogenförmigen Abschnitts mit einem Verbindungsabschnitt vorgesehen ist.

5. Interventionelle künstliche Herzklappe nach Anspruch 1, wobei der Stützabschnitt eine erste Reihe von Stützabschnitten und eine zweite Reihe von Stützabschnitten umfasst, die in Umfangsrichtung angeordnet sind, wobei die erste Reihe von Stützabschnitten näher an der Seite des Einströmendes als die zweite Reihe von Stützabschnitten ist.

6. Interventionelle künstliche Herzklappe nach Anspruch 5, wobei
der bogenförmige Abschnitt in einem Bereich angeordnet ist, der im Wesentlichen von der ersten Reihe von Stützabschnitten umschlossen ist; und
der Hilfsabschnitt in einem Bereich angeordnet ist, der im Wesentlichen von der ersten Reihe von Stützabschnitten oder der zweiten Reihe von Stützabschnitten umschlossen ist.

7. Interventionelle künstliche Herzklappe nach Anspruch 6, wobei der Hilfsabschnitt einen bogenförmigen Hilfsabschnitt und an zwei Enden des Hilfsabschnitts angeordnete Verbindungsabschnitte umfasst, und die Verbindungsabschnitte mit dem bogenförmigen Abschnitt verbunden sind; und der Hilfsabschnitt in einem Bereich angeordnet ist, der im Wesentlichen von der ersten Reihe von Stützabschnitten umschlossen ist.

8. Interventionelle künstliche Herzklappe nach Anspruch 6, wobei der bogenförmige Abschnitt eine Vielzahl von getrennten Abschnitten umfasst, und der Hilfsabschnitt eine Vielzahl von getrennten Abschnitten umfasst; die getrennten Abschnitte des bogenförmigen Abschnitts und die getrennten Abschnitte des Hilfsabschnitts durch eine Vielzahl von Verbindungsabschnitten miteinander verbunden sind; der bogenförmige Abschnitt, der Hilfsabschnitt und die Verbindungsabschnitte hohle röhrenförmige Elemente sind; und der Hilfsabschnitt in einem Bereich angeordnet ist, der im Wesentlichen von der ersten Reihe von Stützabschnitten umschlossen ist.

9. Interventionelle künstliche Herzklappe nach Anspruch 6, wobei der Hilfsabschnitt einen bogenförmigen Hilfsabschnitt und an zwei Enden des Hilfsabschnitts angeordnete Verbindungsabschnitte umfasst, und die Verbindungsabschnitte mit dem bogenförmigen Abschnitt verbunden sind; und der Hilfsabschnitt in einem Bereich angeordnet ist, der im Wesentlichen von der zweiten Reihe von Stützabschnitten umschlossen ist.

10. Interventionelle künstliche Herzklappe nach Anspruch 5, wobei die erste Reihe von Stützabschnitten und/oder die zweite Reihe von Stützabschnitten nahe bei jedem der zwei Verbindungselemente einen konvergenten Abschnitt aufweisen, und ein Stützabschnitt des konvergenten Abschnitts in radialer Richtung stärker konvergent als andere Stützabschnitte der ersten Reihe von Stützabschnitten und der zweiten Reihe von Stützabschnitten ist.

11. Interventionelle künstliche Herzklappe nach Anspruch 1, wobei ein erstes Verbindungselement und ein zweites Verbindungselement am Rand des Körperabschnitts an der Seite des Ausströmendes vorgesehen sind, wobei das erste Verbindungselement mit einer ersten Verbindungssäule und einer zweiten Verbindungssäule vorgesehen ist, und das zweite Verbindungselement mit einer dritten Verbindungssäule und einer vierten Verbindungssäule vorgesehen ist;
das Verankerungsteil ein erstes Verankerungselement und ein zweites Verankerungselement umfasst, wobei ein Verbindungsabschnitt des ersten Verankerungselements eine erste Buchse und eine zweite Buchse umfasst, und ein Verbindungsabschnitt des zweiten Verankerungselements eine dritte Buchse und eine vierte Buchse umfasst; und
die erste Buchse, die zweite Buchse, die dritte Buchse und die vierte Buchse die erste Verbindungssäule, die dritte Verbindungssäule, die zweite Verbindungssäule und die vierte Verbindungssäule jeweils aufnehmen können.

12. Interventionelle künstliche Herzklappe nach Anspruch 1, wobei der Klappenstent in einem radial komprimierten Zustand in einem Katheter angeordnet werden kann, und das Verankerungsteil Elastizität aufweist und nach elastischer Verformung in einem Katheter angeordnet werden kann.

13. Interventionelle künstliche Herzklappe nach einem der Ansprüche 1-11 oder medizinische Einrichtung nach Anspruch 12, wobei der Körperabschnitt nahe dem Ausströmende einen nach innen zurückgezogenen Bereich aufweist.

14. Interventionelle künstliche Herzklappe nach einem der Ansprüche 1-11 oder medizinische Einrichtung nach Anspruch 12, wobei der Schürzenabschnitt eine trichterförmige Struktur aufweist, der Körperabschnitt im Wesentlichen zylindrisch ist, und ein Ende mit kleinerem Durchmesser der trichterförmigen Struktur des Schürzenabschnitts mit dem Körperabschnitt verbunden ist;
der Umfangsrand der trichterförmigen Struktur eine nach unten abgekantete Struktur aufweist, um eine Wirkung der Verankerung an einem nativen Klappenring zu verstärken.

## Revendications

1. Prothèse (500) valvulaire cardiaque interventionnelle, comprenant une endoprothèse valvulaire (2) et une partie d'ancrage, dans laquelle l'endoprothèse valvulaire (2) présente une section de corps (10) et une section de jupe (120) au niveau d'une extrémité d'entrée ; une partie de feuillet valvulaire est agencée dans la section de corps (10) ; l'endoprothèse valvulaire (2) peut être radialement aplatie et dilatée entre une configuration radialement aplatie et une configuration radialement dilatée ; au moins une partie de la surface externe de la section de jupe (120) est recouverte par un élément (15) d'étanchéité annulaire flexible, l'élément (15) d'étanchéité annulaire flexible comprend un matériau en mesure d'absorber le sang ; le matériau en mesure d'absorber le sang se dilate après avoir absorbé du sang ; au moins deux éléments de liaison (17) sont agencés au niveau d'un côté d'une extrémité de sortie de la section de corps (10) ; et la partie d'ancrage est agencée autour de la périphérie de l'endoprothèse valvulaire (2) et reliée de manière détachable aux éléments de liaison (17) ; la partie d'ancrage comprend au moins deux éléments d'ancrage, dans laquelle l'élément d'ancrage présente une section en arc (23), une section auxiliaire (25) et une partie de liaison, dans laquelle la partie de liaison est prévue au niveau d'une extrémité libre de la section en arc (23), et la partie de liaison peut être reliée de manière détachable aux éléments de liaison (17) ; et la section auxiliaire (25) est reliée à la section en arc (23) pour maintenir la valve cardiaque dans le corps humain ; dans laquelle la partie d'ancrage peut être reliée à l'élément de liaison, et est agencée autour de la périphérie de l'endoprothèse valvulaire (2) après avoir été reliée à l'élément de liaison, et la section auxiliaire (25) est agencée près de l'extrémité de sortie et près du côté de la section de corps (10) par rapport à la section en arc (23) ; dans laquelle la section de corps (10) comprend une pluralité de parties de support (11) agencées circonférentiellement ; et les parties de support (11) s'étendent radialement vers l'extérieur et vers l'extrémité d'entrée.

2. Prothèse valvulaire cardiaque interventionnelle selon la revendication 1, dans laquelle l'élément d'étanchéité annulaire flexible recouvre en outre une zone partielle de la section de corps au niveau du côté de l'extrémité d'entrée.

3. Prothèse valvulaire cardiaque interventionnelle selon la revendication 1, dans laquelle la section de corps comprend une partie de maintien, la partie de maintien est agencée au niveau du bord de la section de corps au niveau du côté de l'extrémité d'entrée pour empêcher l'élément d'étanchéité annulaire flexible de pénétrer dans un espace interne à l'intérieur de l'endoprothèse valvulaire.

4. Prothèse valvulaire cardiaque interventionnelle selon la revendication 1, dans laquelle l'élément d'ancrage présente une section en arc, une extrémité libre de la section en arc étant prévue avec une partie de liaison.

5. Prothèse valvulaire cardiaque interventionnelle selon la revendication 1, dans laquelle la partie de support comprend une première bande de partie de support et une deuxième bande de partie de support qui sont agencées dans la direction circonférentielle, dans laquelle la première bande de partie de support est plus proche du côté de l'extrémité d'entrée que la deuxième bande de partie de support.

6. Prothèse valvulaire cardiaque interventionnelle selon la revendication 5, dans laquelle
la section en arc se situe dans une zone qui est généralement entourée par la première bande de partie de support ; et
la section auxiliaire se situe dans une zone qui est généralement entourée par la première bande de partie de support ou la deuxième bande de partie de support.

7. Prothèse valvulaire cardiaque interventionnelle selon la revendication 6, dans laquelle la section auxiliaire comprend une section auxiliaire en arc et des sections de liaison agencées au niveau des deux extrémités de la section auxiliaire, et les sections de liaison sont reliées à la section en arc ; et la section auxiliaire se situe dans une zone qui est généralement entourée par la première bande de partie de support.

8. Prothèse valvulaire cardiaque interventionnelle selon la revendication 6, dans laquelle la section en arc comprend une pluralité de sections séparées, et la section auxiliaire comprend une pluralité de sections séparées ; les sections séparées de la section en arc et les sections séparées de la section auxiliaire sont reliées ensemble par une pluralité de sections de liaison ; la section en arc, la section auxiliaire et les sections de liaison sont des éléments tubulaires creux ; et la section auxiliaire se situe dans une zone qui est généralement entourée par la première bande de partie de support.

9. Prothèse valvulaire cardiaque interventionnelle selon la revendication 6, dans laquelle la section auxiliaire comprend une section auxiliaire en arc et des sections de liaison agencées au niveau des deux extrémités de la section auxiliaire, et les sections de liaison sont reliées à la section en arc ; et la section auxiliaire se situe dans une zone qui est généralement entourée par la deuxième bande de partie de support.

10. Prothèse valvulaire cardiaque interventionnelle selon la revendication 5, dans laquelle la première bande de partie de support et/ou la deuxième bande de partie de support présentent une partie convergente près de chacun des deux éléments de liaison, et une partie de support de la partie convergente est plus convergente radialement que d'autres parties de support de la première bande de partie de support et de la deuxième bande de partie de support.

11. Prothèse valvulaire cardiaque interventionnelle selon la revendication 1, dans laquelle un premier élément de liaison et un deuxième élément de liaison sont prévus au niveau du bord de la section de corps au niveau du côté de l'extrémité de sortie, le premier élément de liaison est prévu avec une première colonne de liaison et une deuxième colonne de liaison, et le deuxième élément de liaison est prévu avec une troisième colonne de liaison et une quatrième colonne de liaison ;
la partie d'ancrage comprend un premier élément d'ancrage et un deuxième élément d'ancrage, une partie de liaison du premier élément d'ancrage comprend une première douille et une deuxième douille, et une partie de liaison du deuxième élément d'ancrage comprend une troisième douille et une quatrième douille ; et
la première douille, la deuxième douille, la troisième douille et la quatrième douille peuvent recevoir respectivement la première colonne de liaison, la troisième colonne de liaison, la deuxième colonne de liaison et la quatrième colonne de liaison.

12. Prothèse valvulaire cardiaque interventionnelle selon la revendication 1, dans laquelle l'endoprothèse valvulaire peut être placée dans un cathéter dans un état radialement aplati, et la partie d'ancrage présente une élasticité et peut être placée dans un cathéter après déformation élastique.

13. Prothèse valvulaire cardiaque interventionnelle selon l'une quelconque des revendications 1 à 11 ou appareil médical selon la revendication 12, dans laquelle ou lequel la section de corps présente une zone rétractée vers l'intérieur près de l'extrémité de sortie.

14. Prothèse valvulaire cardiaque interventionnelle selon l'une quelconque des revendications 1 à 11 ou appareil médical selon la revendication 12, dans laquelle la section de jupe présente une structure en forme de trompette, la section de corps est généralement cylindrique, et une extrémité de plus petit diamètre de la structure en forme de trompette de la section de jupe est reliée à la section de corps ;
le bord périphérique de la structure en forme de trompette présente une structure à rebord dirigée vers le bas pour renforcer un effet d'ancrage sur un anneau valvulaire d'origine.
